# EUROPEAN PATENT SPECIFICATION

(11) **EP 2 291 081 B1**
(45) Date of publication and mention of the grant of the patent: **03.06.2020**
(21) Application number: 09762915.8
(22) Date of filing: 10.06.2009
(51) Int. Cl.: A01N 45/00, A61K 31/56, A61K 9/08, A61K 31/79

(54) **POVIDONE IODINE, A NOVEL ALTERNATIVE PRESERVATIVE FOR OPHTHALMIC COMPOSITIONS**
POVIDON-IOD, EIN NEUES ALTERNATIVES KONSERVIERUNGSMITTEL FÜR OPHTHALMISCHE ZUSAMMENSETZUNGEN
LA POVIDONE IODÉE, NOUVEAU CONSERVATEUR INNOVANT POUR COMPOSITIONS OPHTALMIQUES

(30) Priority: 15.12.2008 US 201854 P; 12.06.2008 US 129222 P
(43) Date of publication of application: 09.03.2011
(73) Proprietor: Foresight Biotherapeutics, Inc., New York, NY 10019 (US)
(72) Inventor: CAPRIOTTI, Joseph, A., New York NY 10025 (US); LIANG, Bo, East Brunswick NJ 08816 (US); SAMSON, C., Michael, New York NY 10016 (US); STEIN, Jason, New York NY 10162 (US); WEISER, Michael, New York NY (US)
(74) Representative: Carpmaels & Ransford LLP
(86) International application number: PCT/US2009/003521
(87) International publication number: WO 2009/151619

(56) References cited:
- WO-A2-2007/106381
- US-A- 5 126 127
- US-A1- 2007 219 170
- US-A1- 2007 297 990
- US-B1- 6 328 991

## Description

### BACKGROUND

Preservatives are currently an integral part of ophthalmic preparations. Suitable antimicrobial preservatives are typically added to prevent multi-dose package contamination. Such agents may include benzalkonium chloride, thimerosal, chlorobutanol, methyl paraben, propyl paraben, phenylethyl alcohol, EDTA, sorbic acid, Onamer M, other agents known to those skilled in the art, or a combination thereof. Benzalkonium chloride (also referred to as BAK or BAC) is the most common preservative used in ophthalmic preparations. Typically, such preservatives are employed at a level of from 0.001% to 1.0% by weight. However, recent evidence highlight the potential corneal and conjunctival toxicity of BAK, which lead to serious medical problems such as ocular medicamentosa, decreased success of glaucoma surgery, or reduced compliance with ocular medications.

Topical ophthalmic compositions comprising povidone-iodine combined with a steroid or non-steroidal anti-inflammatory drug are described in WO 2007/106381 A2.

### BRIEF SUMMARY OF THE INVENTION

Embodiments of the present invention are set out in the enclosed claims.

The present invention provides a stable ophthalmic composition suitable for topical administration to the eye comprising a mixture of:
a. Povidone-iodine (PVP-I) at a concentration between 0.01% and 10%, and
b. a steroid, wherein the steroid is dexamethasone, and wherein the pH of the composition is in the range of 3 to 4.

Disclosed herein is a preserved ophthalmic preparation comprising povidone-iodine (PVP-I) at a concentration sufficient to preserve the ophthalmic preparation, and at least one member selected from the group consisting of a steroidal anti-inflammatory compound, a non-steroidal anti-inflammatory compound, an antibacterial compound, an anti-allergy compound, and an anti-glaucoma compound.

In an embodiment, PVP-I is present at a concentration selected from the group consisting of 0.01% to 10%, 0.1% to 2.5%, 0.2 to 1.5%, 0.3% to 1.0%. In another embodiment, PVP-I is present at a concentration of 0.5%.

In an embodiment, the PVP-I concentration is measured on a weight/weight basis with respect to the overall preparation. In another embodiment, the PVP-I concentration is measured on a weight/volume basis with respect to the overall preparation.

In an embodiment, a preparation includes an anti-inflammatory compound selected from the group consisting of ketotifen fumarate, diclofenac sodium, nepafenac, bromfenac, flurbiprofen sodium, suprofen, celecoxib, naproxen, rofecoxib, and any combination thereof.

In an embodiment of the disclosure, a preparation includes an anti-inflammatory compound selected from the group consisting of dexamethasone, dexamethasone alcohol, dexamethasone sodium phosphate, fluromethalone acetate, fluromethalone alcohol, lotoprendol etabonate, medrysone, prednisolone acetate, prednisolone sodium phosphate, difluprednate, rimexolone, hydrocortisone, hydrocortisone acetate, lodoxamide tromethamine, and any combination thereof.

In an embodiment, a preparation further includes an antimicrobial preservative selected from the group consisting of benzalkonium chloride, thimerosal, chlorobutanol, methyl paraben, propyl paraben, phenylethyl alcohol, EDTA, sorbic acid, Onamer M and any combination thereof.

In an embodiment, a preparation further includes a viscosity increasing agent. In an embodiment, a viscosity increasing agent is selected from the group consisting of polyvinyl alcohol, polyvinylpyrrolidone, methyl cellulose, hydroxypropylmethylcellulose, hydroxyethylcellulose, carboxymethylcellulose, hydroxypropylcellulose, and any combination thereof.

In an embodiment, a preparation further com includes prises at least one artificial tears-based lubricant.

The disclosure also includes a method of preserving an ophthalmic preparation, the method comprising adding to an ophthalmic preparation PVP-I in an amount sufficient to preserve the ophthalmic preparation. In an embodiment of the disclosure, the method includes adding PVP-I to exist at a concentration selected from the group consisting of 0.01% to 10%,0.1% to 2.5%, 0.2 to 1.5%, and 0.3% to 1.0%. In an embodiment of the disclosure, PVP-I is present at a concentration of 0.5%.

### DETAILED DESCRIPTION

As used herein, the term "about" means plus or minus 10% of a referenced value, inclusive of the value.

Ranges set forth herein are intended to include every value between the two referenced endpoints, inclusive of the endpoint values.

Herein, it is now shown that povidone iodine can serve as a preservative for variety of pharmaceutical compositions. In an aspect of the invention, povidone iodine is shown to be a preservative for ophthalmic preparations. Therefore, in an embodiment, the invention provides a preserved ophthalmic composition comprising a povidone iodine composition.

In an embodiment, povidone iodine functions as at least a preservative for variety of pharmaceutical compositions. In an embodiment, povidone iodine is a preservative in an ophthalmic composition. The concentration of povidone-iodine as a preservative in ophthalmic compositions can range from 0.01% - 10% (weight/weight or weight/volume), and all concentrations in between. In an embodiment, the povidone-iodine concentration is between 0.1% and 2.5%, in another embodiment, between 0.2 and 1.5%, and in yet another embodiment, between 0.3% and 1.0%. In an embodiment, the povidone-iodine concentration is about 0.5%.

In an aspect of the invention, povidone iodine provides an antimicrobial property to an ophthalmic preparation. In another aspect, povidone iodine provides an ophthalmic preparation with one or more non-antimicrobial preservative properties (e.g., antioxidant).

In an embodiment, the disclosure also provides povidone-iodine compositions comprising one or more components in addition to the povidone-iodine component, as set forth herein.

In an aspect, the disclosure provides a broad spectrum of povidone-iodine ophthalmic compositions, for example, comprising povidone iodine as a preservative to be used in cases of ocular conjunctival or corneal infection caused by mycobacteria, viruses, fungi, and amoeba. In another aspect, compositions of the disclosure are useful in the infectious prophylaxis of patients recovering from recent ophthalmic surgery, among other ophthalmic procedures.

In various embodiments, povidone-iodine ophthalmic compositions according to the disclosure include, but are not limited to the following:
1. Artificial-tears preparations comprising povidone iodine as a preservative. Artificial-tear based constituents include, but are not limited to, ophthalmically-acceptable lubricants, propylene glycol, glycerin, polyethylene glycol, dextran, blended polyvinyl alcohols, polyvinyl alcohol, polyethylene glycol, light mineral oil, hydroxypropyl methylcellulose, hypromellose, carbopol, carbomer 940 (polyacrylic acid), polyvinyl pyrrolidone, white petrolatum, soy lecithin, and sodium carboxyl methylcellulose, as well as other agents known to those skilled in the art, or any combination thereof. Typically, such constituents are employed at a level of from 0.1% to 2% by weight. In an embodiment of the disclosure, the constituents are 1.0% Propylene glycol, 0.3% glycerin, 2.7% blended polyvinyl alcohols, 1% Polyvinyl Alcohol, 1% Polyethylene Glycol, light mineral oil, 0.3% hydroxypropyl methylcellulose, 1.0% Soy lecithin, 0.25% or 0.5% sodium carboxyl methylcellulose. In another embodiment of the disclosure, the total weight of the PVP-I, artificial-tear based constituents is between 0.1% and 4.5% of the total weight of the composition.
2. Anti-inflammatory and steroid preparations comprising povidone iodine as a preservative. Non-limiting examples of suitable anti-inflammatories include: ketotifen fumarate, diclofenac sodium, nepafenac, bromfenac, flurbiprofen sodium, suprofen, celecoxib, naproxen or rofecoxib. Non-limiting examples of suitable steroids include: Dexamethasone alcohol, dexamethasone sodium phosphate, fluromethalone acetate, fluromethalone alcohol, lotoprendol etabonate, medrysone, prednisolone acetate, prednisolone sodium phosphate, difluprednate, rimexolone, hydrocortisone, hydrocortisone acetate, lodoxamide tromethamine. The anti-inflammatory and steroid constituents are typically used at a concentration of 0.01% to 2.0% by weight of the total composition. In an aspect, about 0.1% dexamethasone is used in a preserved ophthalmic preparation.
3. BAK-free ophthalmic compositions to treat glaucoma comprising povidone iodine as a preservative. Non-limiting examples of suitable glaucoma medicines include: Beta Blockers (levobunolol, timolol hemihydrate, betaxolol hydrochloride, timolol maleate, and related salts thereof); prostaglandin analogs (for example bimatoprost, travoprost, Latanoprost); Alpha Agonists (brimonidine, Iopidine, apraclonidine); Carbonic Anhydrase Inhibitors (brinzolamide, dorzolamide). Such constituents are used at concentrations typically used in the art.
4. Antibiotic/Antimicrobial ophthalmics comprising povidone iodine as a preservative. Non-limiting examples of suitable include fluoroquinolones (ciprofloxacin, levofloxacin, ofloxacin, moxifloxacin, gatifloxacin, etc...); Aminoglycosides (tobramycin, gentamicin, neomycin, etc...); Polymyxin B Combinations (polymyxin B/trimethoprim, Polysporin polymyxin B/bacitracin Neosporin polymyxin B/neomycin/gramicidin, etc.) and other antibiotics (azithromycin, ilotycin, erythromycin, bacitracin, etc...). Typically, such antibiotic and antimicrobial constituents are employed at a level of from 0.001% to 1.0% by weight of the total composition.
5. Anti-Allergic preparations comprising povidone iodine as a preservative. Non-limiting examples of suitable components include epinastine, emedastine difumarate azelastine hydrochloride, olopatadine hydrochloride, olopatadine, ketotifen fumarate, pemirolast potassium, nedocromil, lodoxamide, cromolyn and cromolyn salts. Such constituents are used at concentrations typically used in the art.
6. Multiple preservative ophthalmic preparations comprising povidone iodine as a preservative. Non-limiting examples of suitable components include an antimicrobial preservative. In an embodiment, an antimicrobial preservative is selected from the group consisting of benzalkonium chloride, thimerosal, chlorobutanol, methyl paraben, propyl paraben, phenylethyl alcohol, EDTA, sorbic acid, Onamer M and combinations thereof. Such constituents are used at concentrations typically used in the art.

Additionally, non-limiting examples of suitable excipients for povidone-iodine compositions of the disclosure include co-solvents/surfactants, viscosity-altering agents, and/or bioadhesive agents. Such constituents are used at concentrations typically used in the art.

In an aspect, the compositions of the disclosure may optionally include a co- solvent or surfactant. In an embodiment of the disclosure, a co-solvent may be the same or different than a surfactant. In an embodiment of the disclosure, the solubility of the components of the compositions may be enhanced by inclusion of a surfactant or appropriate co-solvent in the composition. Such co-solvents/surfactants include, but are not limited to, polysorbate-20, -60, and -80, polyoxyethylene /polyoxypropylene surfactants (e.g. Pluronic F-68, F-84 and P-103), cyclodextrin, tyloxapol, PEG 35 Caster oil (Cremophor EL), polyoxyl 40 Stearate (Myrj 52), as well as other agents known to those skilled in the art, or any combination thereof. Typically, such co-solvents are employed at a level of from 0.01% to 2% by weight.

In another aspect, the compositions of the disclosure may optionally comprise an optional viscosity-increasing or viscosity-decreasing agent. Viscosity increased above that of simple aqueous solutions may be desirable to increase ocular absorption of the active compound, to decrease variability in dispensing the formulation, to decrease physical separation of components of a suspension or emulsion of the formulation and/or to otherwise improve the ophthalmic formulation. Such viscosity-enhancing agents include, but are not limited to, polyvinyl alcohol, polyvinylpyrrolidone, methyl cellulose, hydroxypropylmethylcellulose, hydroxyethylcellulose, carboxymethylcellulose, hydroxypropylcellulose, other agents known to those skilled in the art, and/or any combination thereof. Such agents are typically employed at a level of from 0.01% to 2% by weight.

In another aspect, bioadhesive agents may comprise the compositions, in order to increase the retention time of the drug gradient over a biological substrate. The bioadhesive agents include, but are not limited to: polyvinylpyrrolidone (PVP), xanthan gum, locust bean gum, acacia gum, hydroxypropyl methylcellulose (HPMC), sodium alginate, pectin, gelatin, carbomer, polyvinylalcohol, gellan gum, tragacanth, acacia, and sodium carboxymethyl cellulose, as well as other agents known to those skilled in the art, or any combination thereof.

Furthermore, the compositions can be combined with an effective amount of a chemical agent to provide a cooling sensation to relieve mild ocular irritation, enhance ocular comfort, provide a refreshing effect, and improved sensation, when the povidone-iodine solution is applied to the eyes. Such an agent encompasses various chemicals and chemical classes, including, but not limited to, cooling agents such as menthol, menthol derivatives including methone glycerin acetyl and menthyl esters, carboxamides, menthane glycerol ketals, alkyl substituted ureas, sulfonamides, terpene analogs, furanones, and phosphine oxides; or camphor, and bomeol.

The povidone-iodine-comprising composition may be in the form of a solution, a suspension, an emulsion, an ointment, a cream, a gel, or a controlled-release/sustain-release vehicle. For example, the composition may be in the form of a contact lens solution, eyewash, eyedrop, and the like.

In any of the compositions of this disclosure for topical administration, such as topical administration to the eye, the mixtures can be formulated as aqueous solutions at a pH in the range of 3.5 to 6.5. It will be understood that a range listed herein is intended to encompass the upper and lower bounds of the range, inclusively. In an embodiment of the disclosure, the pH is in the range of 4 to 5. This pH range may be achieved by the addition of acids/bases to the solution. In another embodiment of the disclosure, the pH is in the range of 3 to 7.

The disclosure also provides methods of using a povidone-iodine-comprising composition. In an embodiment of the disclosure, the dose volume administered to a subject may be between about 10 microliters and about 200 microliters, in another embodiment of the disclosure, between about 20 microliters and about 100 microliters, and in another embodiment of the disclosure, between about 50 microliters and about 80 microliters, and in another embodiment of the disclosure, about one drop per eye. In an aspect, one drop may be between about 50 and about 80 microliters.

In an embodiment of the disclosure, administration frequency may be anywhere in the range of 1 to 100 times a day. In another embodiment of the disclosure, administration frequency may be between 2 and 24 times a day. In another embodiment of the disclosure, administration frequency may be between 2 and 4 times a day. While the precise regimen can be identified by the skilled artisan, the composition may be topically applied by placing one drop in each eye about 1 to about 24 times daily. For example, the solution may be applied 1, 2, 3, 4, 5, 6, 7, 8, 9, 10, 11, 12, 13, 14, 15, 16, 17, 18, 19, 20, 21, 22, 23, 24, 48, or 96 times a day.

### EXAMPLES

The invention is now described with reference to the following Examples.

### Example 1: Antimicrobial Preservative Effectiveness Tests of PVP-I preserved artificial tears

A composition including at least one artificial tear constituent according to the invention, further comprising PVP-I, may be used as a preservative. The preservative properties of a composition of the invention, such as an ophthalmic preparation comprising PVP-I, can be tested, for example, using USP <51>, or by any method known in the art to determine the effectiveness of an antimicrobial preservative. PVP-I solutions having concentrations of about 0.36% by weight are prepared as set forth herein. By way of a non-limiting example, a PVP-I containing solution is prepared using about 0.36% PVP-I, by weight, as desired in the final product, and combining the PVP-I with 0.01% edentate disodium, 1.2% sodium sulfate, 0.25% hydroxyethylcellulose, 0.5% tyloxapol, 0.35% sodium chloride to adjust osmolality within 300-350 mOsm/kg, sodium hydroxide and/or sulfuric acid to adjust the pH to about 4.0, and enough purified water to bring the total volume up to 100% total.

The test solutions are challenged with known numbers of standard laboratory strains selected. At periodic intervals: 0, 7, 14, and 28 days, samples are removed and assayed to determine survival.

### Example 2: Antimicrobial Preservative Effectiveness Tests of PVP-I preserved 0.1% dexamethasone suspension

The preservative properties of a composition of the invention, such as an ophthalmic preparation comprising PVP-I, can be tested, for example, using USP <51>, or by any method known in the art to determine the effectiveness of an antimicrobial preservative. PVP-I solutions having concentrations of about 0.18% and about 0.36% by weight are prepared as set forth herein. By way of a non-limiting example, a PVP-I containing solution is prepared using 0.18%, or 0.36% PVP-I, by weight, as desired in the final product, and combining the PVP-I with 0.1% dexamethasone, 0.01% edentate disodium, 1.2% sodium sulfate, 0.25% hydroxyethylcellulose, 0.5% tyloxapol, 0.35% sodium chloride to adjust osmolality within 300-350 mOsm/kg, sodium hydroxide and/or sulfuric acid to adjust the pH to 4.0, and enough purified water to bring the total volume up to 100% total. The test solutions are challenged with known numbers of standard laboratory strains selected. At periodic intervals: 0, 7, 14, and 28 days, samples are removed and assayed to determine survival.

### Example 3: Antimicrobial Activity of Preserved PVP-I Solutions

By way of a non-limiting example, PVP-I solutions having concentrations of about 0.36% by weight are prepared as set forth herein. By way of a non-limiting example, a PVP-I containing solution is prepared using 0.36% PVP-I, by weight, as desired in the final product, and combining the PVP-I with 0.1% dexamethasone, 0.01% edentate disodium, 1.2% sodium sulfate, 0.25% hydroxyethylcellulose, 0.5% tyloxapol, 0.35% sodium chloride to adjust osmolality within 300-350 mOsm/kg, sodium hydroxide and/or sulfuric acid to adjust the pH to 4.0, and enough purified water to bring the total volume up to 100% total. These solutions are then tested for *in-vitro* microbiological activity. Microbiological activity can be tested for antimicrobial activity against, for example, bacteria found in the mouth (*P. gingivalis*), or against other bacteria. In another example, killing time tests are conducted with a series of log phase cultures of gram negative and gram positive organisms including Gentamicin resistant *Pseudomonas aeruoinosa, methicilin-resistant staph aureus, E. coli, chlamydia trachoma* and selected viruses. Controls used may include ophthalmic preparations of commercially available antimicrobial products. Bacterial samples are taken at 30 seconds, 1, 2, 5, 10 and 15 minutes and transferred into culture media containing inactivators for iodine. Similarly, virus killing time tests are sampled at one minute and transferred into inactivating media. The results obtained with the experimental samples are compared with the control samples to assess the level of antimicrobial activity of a composition of the invention.

### Example 4: Preparation of PVP-I preserved 0.3% tobramycin and 0.1% dexamethasone suspension

BAK-free Tobradex® ophthalmic suspension preserved with PVP-I concentration ranging from about 0.36% to about 0.6% by weight is prepared as set forth herein. By way of a non-limiting example, a composition is prepared using 0.36%, 0.48%, or 0.6% PVP-I, by weight, as desired in the final product, and combining with 0.3% tobramycin, 0.1% dexamethasone, 0.01% edentate disodium, 1.2% sodium sulfate, 0.25% hydroxyethylcellulose, 0.5% tyloxapol, 0.35% sodium chloride to adjust osmolality within 300-350 mOsm/kg, sodium hydroxide and/or sulfuric acid to adjust the pH to about 4.0, and enough purified water to bring the total volume up to 100% total.

### Example 5: Preparation of PVP-I preserved 0.3% tobramycin solution

BAK-free tobramycin ophthalmic solution preserved with PVP-I in a concentration range of about 0.36% to about 0.6% by weight is prepared as set forth herein. By way of a non-limiting example, an composition is prepared using 0.36%, 0.48%, or 0.6% PVP-I, by weight, as desired in the final product, and combining with 0.3% tobramycin, boric acid, sodium sulfate, sodium chloride, tyloxapol, sodium hydroxide and/or sulfuric acid (to adjust pH) and purified water.

### Example 6: Preparation of PVP-I preserved 0.5% moxifloxacin hydrochloride ophthalmic solution

BAK-free moxifloxacin hydrochloride ophthalmic solution preserved with PVP-I in a concentration range of about 0.36% to about 0.6% by weight is prepared as set forth herein. By way of a non-limiting example, a composition is prepared using 0.36%, 0.48%, or 0.6% PVP-I, by weight, as desired in the final product, and combining with 0.5% moxifloxacin hydrochloride, boric acid, sodium chloride, and purified water. In an embodiment, a preserved ophthalmic solution comprises hydrochloric acid and/or sodium hydroxide to adjust pH.

### Example 7: Preparation of PVP-I preserved 1.0% prednisolone acetate suspension

BAK-free prednisolone acetate suspension preserved with PVP-I in a concentration range of about 0.36% to about 0.6% by weight is prepared as set forth herein. By way of a non-limiting example, a composition is prepared using 0.36%, 0.48%, or 0.6% PVP-I, by weight, as desired in the final product, and combining with 1.0% prednisolone acetate, boric acid, edetate disodium, hypromellose, polysorbate 80, sodium bisulfite, sodium citrate, sodium chloride, and purified water.

## Claims

1. A stable ophthalmic composition suitable for topical administration to the eye, comprising a mixture of:
a. povidone-iodine (PVP-I) at a concentration between 0.01% and 10%, and
b. a steroid, wherein the steroid is dexamethasone, and wherein the pH of the composition is in the range of 3 to 4.

2. The ophthalmic composition of claim 1, wherein the PVP-I is present at a concentration of from 0.1% to 2.5%.

3. The ophthalmic composition of claim 1, wherein the PVP-I is present at a concentration of 0.5%.

4. The ophthalmic composition of claim 2, wherein the PVP-I concentration is measured on a weight/weight basis with respect to the overall preparation.

5. The ophthalmic composition of claim 2, wherein the PVP-I concentration is measured on a weight/volume basis with respect to the overall preparation.

6. The ophthalmic composition of claim 1, wherein said composition further comprises an antimicrobial preservative selected from the group consisting of benzalkonium chloride, thimerosal, chlorobutanol, methyl paraben, propyl paraben, phenylethyl alcohol, EDTA, sorbic acid, Onamer M and any combination thereof.

7. The ophthalmic composition of claim 1, wherein said composition further comprises a viscosity increasing agent.

8. The ophthalmic composition of claim 7, wherein said viscosity increasing agent is selected from the group consisting of polyvinyl alcohol, polyvinylpyrrolidone, methyl cellulose, hydroxypropylmethylcellulose, hydroxyethylcellulose, carboxymethylcellulose, hydroxypropylcellulose, and any combination thereof.

9. The ophthalmic composition of claim 1, wherein the composition comprises at least one artificial tears-based lubricant.

10. The ophthalmic composition of claim 1, wherein said steroid is present at a concentration between 0.01 and 2%.

11. The ophthalmic composition of claim 10, wherein said steroid is present at a concentration of 0.1%.

12. The ophthalmic composition of claim 1, wherein said composition further comprises a co-solvent/surfactant.

13. The ophthalmic composition of claim 12, wherein said co-solvent/surfactant is selected from the group consisting of polysorbate 20, polysorbate 60, polysorbate 80, Pluronic F-68, Pluronic F-84, Pluronic P-103, cyclodextrin, tyloxapol and a combination thereof.

14. The ophthalmic composition of claim 12, wherein said co-solvent/surfactant is at a concentration of about 0.01% to 2% by weight in said composition.

15. The ophthalmic composition of any one of claims 1-14, wherein said pH is pH 4.

## Patentansprüche

1. Stabile, für die topische Anwendung am Auge geeignete ophthalmische Zusammensetzung, umfassend eine Mischung aus:
a. Povidon-Iod (PVP-I) in einer Konzentration zwischen 0,01% und 10% und
b. ein Steroid, wobei es sich bei dem Steroid um Dexamethason handelt, und wobei der pH-Wert der Zusammensetzung im Bereich von 3 bis 4 liegt.

2. Ophthalmische Zusammensetzung nach Anspruch 1, wobei das PVP-I in einer Konzentration von 0,1% bis 2,5% vorliegt.

3. Ophthalmische Zusammensetzung nach Anspruch 1, wobei das PVP-I in einer Konzentration von 0,5% vorliegt.

4. Ophthalmische Zusammensetzung nach Anspruch 2, wobei die PVP-I-Konzentration auf Gewicht/Gewicht-Basis, bezogen auf die Gesamtzubereitung, gemessen wird.

5. Ophthalmische Zusammensetzung nach Anspruch 2, wobei die PVP-I-Konzentration auf einer Gewicht/Volumen-Basis, bezogen auf die Gesamtzubereitung, gemessen wird.

6. Ophthalmische Zusammensetzung nach Anspruch 1, wobei die Zusammensetzung weiterhin einen antimikrobiellen Konservierungsstoff ausgewählt aus der Gruppe bestehend aus Benzalkoniumchlorid, Thimerosal, Chlorbutanol, Methylparaben, Propylparaben, Phenylethylalkohol, EDTA, Sorbinsäure, Onamer M und einer beliebigen Kombination davon umfasst.

7. Ophthalmische Zusammensetzung nach Anspruch 1, wobei die Zusammensetzung weiterhin ein viskositätserhöhendes Mittel umfasst.

8. Ophthalmische Zusammensetzung nach Anspruch 7, wobei das viskositätserhöhende Mittel aus der aus Polyvinylalkohol, Polyvinylpyrrolidon, Methylcellulose, Hydroxypropylmethylcellulose, Hydroxyethylcellulose, Carboxymethylcellulose, Hydroxypropylcellulose und einer beliebigen Kombination davon bestehenden Gruppe ausgewählt ist.

9. Ophthalmische Zusammensetzung nach Anspruch 1, wobei die Zusammensetzung mindestens ein auf künstlichen Tränen basierendes Schmiermittel umfasst.

10. Ophthalmische Zusammensetzung nach Anspruch 1, wobei das Steroid in einer Konzentration zwischen 0,01 und 2% vorliegt.

11. Ophthalmische Zusammensetzung nach Anspruch 10, wobei das Steroid in einer Konzentration von 0,1% vorliegt.

12. Ophthalmische Zusammensetzung nach Anspruch 1, wobei die Zusammensetzung weiterhin ein Cosolvens/Tensid umfasst.

13. Ophthalmische Zusammensetzung nach Anspruch 12, wobei das Cosolvens/Tensid aus der aus Polysorbat 20, Polysorbat 60, Polysorbat 80, Pluronic F-68, Pluronic F-84, Pluronic P-103, Cyclodextrin, Tyloxapol und einer Kombination davon bestehenden Gruppe ausgewählt ist.

14. Ophthalmische Zusammensetzung nach Anspruch 12, wobei das Cosolvens/Tensid in einer Konzentration von etwa 0,01 Gew.-% bis 2 Gew.-% in der Zusammensetzung vorliegt.

15. Ophthalmische Zusammensetzung nach einem der Ansprüche 1-14, wobei der pH-Wert pH 4 ist.

## Revendications

1. Composition ophtalmique stable appropriée pour une administration topique à l'œil, comprenant un mélange de :
a. povidone-iode (PVP-I) à raison d'une concentration comprise entre 0,01 % et 10 %, et
b. un stéroïde, le stéroïde étant la dexaméthasone, et le pH de la composition se situant dans la plage de 3 à 4.

2. Composition ophtalmique selon la revendication 1, la PVP-I étant présente à raison d'une concentration allant de 0,1 % à 2,5 %.

3. Composition ophtalmique selon la revendication 1, la PVP-I étant présente à raison d'une concentration de 0,5 %.

4. Composition ophtalmique selon la revendication 2, la concentration en PVP-I étant mesurée sur une base poids/poids par rapport à la préparation globale.

5. Composition ophtalmique selon la revendication 2, la concentration en PVP-I étant mesurée sur une base poids/volume par rapport à la préparation globale.

6. Composition ophtalmique selon la revendication 1, ladite composition comprenant en outre un conservateur antimicrobien choisi dans le groupe constitué par le chlorure de benzalkonium, le thimérosal, le chlorobutanol, le méthyleparabène, le propylparabène, l'alcool phényléthylique, l'EDTA, l'acide sorbique, l'Onamer M et une quelconque combinaison correspondante.

7. Composition ophtalmique selon la revendication 1, ladite composition comprenant en outre un agent d'augmentation de la viscosité.

8. Composition ophtalmique selon la revendication 7, ledit agent d'augmentation de la viscosité étant choisi dans le groupe constitué par le poly(alcool vinylique), la polyvinylpyrrolidone, la méthylcellulose, l'hydroxypropylméthylcellulose, l'hydroxyéthylcellulose, la carboxyméthylcellulose, l'hydroxypropylcellulose, et une quelconque combinaison correspondante.

9. Composition ophtalmique selon la revendication 1, la composition comprenant au moins un lubrifiant artificiel à base de larmes.

10. Composition ophtalmique selon la revendication 1, ledit stéroïde étant présent à raison d'une concentration comprise entre 0,01 et 2 %.

11. Composition ophtalmique selon la revendication 10, ledit stéroïde étant présent à raison d'une concentration de 0,1 %.

12. Composition ophtalmique selon la revendication 1, ladite composition comprenant en outre un cosolvant/tensioactif.

13. Composition ophtalmique selon la revendication 12, ledit cosolvant/tensioactif étant choisi dans le groupe constitué par le polysorbate 20, le polysorbate 60, le polysorbate 80, le Pluronic F-68, le Pluronic F-84, le Pluronic P-103, une cyclodextrine, le tyloxapol et une combinaison correspondante.

14. Composition ophtalmique selon la revendication 12, ledit cosolvant/tensioactif étant présent à raison d'une concentration d'environ 0,01 % à 2 % en poids dans ladite composition.

15. Composition ophtalmique selon l'une quelconque des revendications 1 à 14, ledit pH étant pH 4.
